# FASCICULE DE BREVET EUROPEEN

(11) **EP 0 847 979 B1**
(45) Date de publication et mention de la délivrance du brevet: **31.10.2001**
(21) Numéro de dépôt: 97420228.5
(22) Date de dépôt: 09.12.1997
(51) Int. Cl.: C07C 51/487, C07C 51/27, C07C 55/14

(54) **Procédé de préparation de diacides à partir des eaux de lavage des produits d'oxydation du cyclohexane**
Herstellung von Dicarbonsäuren aus Waschwässern von Oxidationsprodukten von Cyclohexan
Process for the preparation of dicarboxylic acids from the wash waters of cyclohexane oxidation

(30) Priorité: 12.12.1996 FR 9615524
(43) Date de publication de la demande: 17.06.1998
(73) Titulaire: RHODIA POLYAMIDE INTERMEDIATES, 69190 Saint-Fons (FR)
(72) Inventeur: Le Bris, Louis, 69005 Lyon (FR)
(74) Mandataire: Esson, Jean-Pierre

(56) Documents cités:
- FR-A- 1 334 673
- FR-A- 2 061 956
- FR-A- 2 219 144

## Description

La présente invention conceme un procédé de préparation de diacides ou de mélanges de diacides à partir des solutions aqueuses provenant du lavage des produits d'oxydation du cyclohexane.

Il est connu d'oxyder le cyclohexane, essentiellement en cyclohexanone et cyclohexanol, par des mélanges gazeux contenant de l'oxygène moléculaire, en présence ou non de catalyseur.

Ainsi lors de l'oxydation du cyclohexane par l'air, en phase liquide, sans catalyseurs, on obtient principalement du cyclohexanol et de la cyclohexanone. Il est connu également dans ce type de procédé d'éliminer, avant de distiller ces deux composés, au moins une partie des produits secondaires formés au cours de la réaction d'oxydation. Cette élimination est habituellement effectuée par lavage à l'eau et/ou à l'aide de solutions aqueuses alcalines, soit à la fin de la réaction d'oxydation, soit pendant ou entre les différentes phases de l'oxydation.

Les produits contenus dans ces eaux de lavage sont notamment des peroxydes, en particulier des peroxydes d'acides, principalement l'acide hydroperoxy-6 hexanoïque et les polymères qui en dérivent.

Ces eaux de lavage contiennent également, de manière non exclusive, de l'acide hydroxycaproïque libre ou estérifié, de l'acide adipique, de l'acide glutarique et de l'acide succinique, ainsi que l'acide formyl-5 valérique.

Il a été proposé divers procédés de valorisation de ces eaux de lavage, notamment en acide adipique.

Ainsi le brevet FR-A-2 061 956 décrit un procédé de préparation d'acide adipique par oxydation, à l'aide d'oxygène moléculaire sous pression, de l'acide hydroperoxy-6 hexanoïque en solution aqueuse. Dans le brevet FR-A-1 594 895 et dans son premier certificat d'addition FR-A-2 054 701, est décrit un procédé de préparation d'acide adipique, par oxydation à l'aide d'acide nitrique, éventuellement en présence de peroxyde d'azote, de l'acide hydroperoxy-6 hexanoïque contenu dans la solution aqueuse de lavage.

Il a également été décrits des procédés d'hydrogénation des acides contenus dans les eaux de lavage en alcools correspondants.

Ainsi dans le brevet US 3 985 814, on a décrit un procédé d'hydrogénation des acides contenus dans les eaux de lavage en alcools correspondants, en présence de platine déposé sur noir de carbone, sous une pression de 300 bar et à 265°C. Ce procédé semble relativement efficace, puisque le taux de conversion des acides en alcools est d'environ 95 %, mais les conditions d'hydrogénation sont très dures, ce qui rend le procédé trop onéreux pour une utilisation industrielle. Par ailleurs, ce procédé par définition transforme les acides en alcools, ce qui ne correspond pas à la valorisation recherchée en diacides carboxyliques.

Un procédé basé sur les mêmes réactions, mis en oeuvre dans des conditions analogues de température et de pression, mais utilisant un catalyseur choisi parmi le ruthénium, le rhénium, le cobalt, le nickel et le chrome, est décrit dans le brevet allemand DE-A-1 957 396.

Enfin le brevet français FR 1 585 375 décrit un procédé de préparation d'acide ε-hydroxy-hexanoïque par hydrogénation catalytique de l'acide hydroperoxy-6 hexanoïque, qui doit être préalablement extrait et séparé des eaux de lavage, au moyen d'un solvant organique.

Ces procédés connus permettent certes d'obtenir des diacides, notamment de l'acide adipique, ou leurs intermédiaires alcooliques que l'on peut éventuellement oxyder, mais ils présentent tous néanmoins au moins l'un des inconvénients suivants :
- leur mise en oeuvre industrielle est trop onéreuse, en raison des conditions dans lesquelles les réactions doivent être mises en jeu ;
- l'acide adipique ainsi obtenu est d'une pureté insuffisante pour être utilisé directement comme intermédiaire de synthèse de fibres polyamides, ce qui nécessite un traitement ultérieur de purification trop important ;
- ces procédés ne traitent qu'une partie des eaux de lavage et non leur totalité.

La but de la présente invention est donc notamment de fournir un procédé de préparation de diacides aliphatiques, notamment d'acide adipique et de mélanges d'acides adipique, glutarique et succinique, à partir des eaux de lavage provenant d'un procédé d'oxydation du cyclohexane et contenant des peroxydes, ledit procédé de préparation de diacides ne présentant pas les inconvénients indiqués précédemment.

Le procédé de l'invention permet en particulier d'obtenir de l'acide adipique ayant une pureté suffisante pour être utilisé comme intermédiaire de synthèse du polyamide-6,6, notamment pour les fibres textiles, ainsi que des mélanges des acides adipique, glutarique et succinique de bonne qualité, tout en étant un procédé peu onéreux à mettre en oeuvre industriellement et permettant de traiter la totalité des composés contenus dans les eaux de lavage.

Un autre but de la présente invention est de fournir un procédé dans lequel les composés peroxydés, contenus dans les eaux de lavage évoquées précédemment, sont décomposés sélectivement et complètement dans des conditions de température et de pression modérées, alors que leur élimination dans les procédés antérieurs conduit à la formation d'impuretés qui se retrouvent dans les diacides obtenus et en particulier dans l'acide adipique.

Plus précisément, la présente invention consiste en un procédé de préparation de diacides aliphatiques à partir des eaux de lavage provenant d'un procédé d'oxydation du cyclohexane et contenant des peroxydes, caractérisé en ce que l'on effectue successivement les étapes suivantes :
a) - on déperoxyde au moins partiellement lesdites eaux de lavage, en les soumettant à une hydrogénation catalytique à une température comprise entre 0°C et 100°C, en présence d'au moins un métal choisi dans le groupe du platine ;
b) - on oxyde à l'aide d'acide nitrique les produits contenus dans les eaux de lavage après déperoxydation.

Par métal du groupe du platine, on entend dans le présent texte le platine, le palladium, le rhodium, le ruthénium, l'iridium et l'osmium.

Il est nécessaire d'effectuer tout d'abord la déperoxydation selon l'étape a) avant l'oxydation nitrique selon l'étape b), sinon on formerait au cours de l'oxydation nitrique des impuretés que l'on retrouverait dans les diacides en fin de procédé.

L'hydrogénation catalytique utilisée pour la déperoxydation de l'étape a) est effectuée de préférence à une température de 20°C à 80°C.

Entre les étapes a) et b), on peut avantageusement effectuer une concentration de l'hydrogénat obtenu en a), par élimination d'une partie de l'eau qu'il contient.

Les eaux de lavage auxquelles s'appliquent le procédé de l'invention peuvent provenir de tout procédé d'oxydation du cyclohexane par l'air, l'air enrichi ou l'air appauvri en oxygène, dans la mesure où lesdites eaux de lavage contiennent des peroxydes. Elles peuvent plus particulièrement provenir du procédé décrit dans le brevet FR 1 580 206.

Dans ces procédés d'oxydation du cyclohexane, les oxydats sont lavés selon toutes les techniques conventionnelles de lavage en phase liquide, l'opération pouvant être conduite en continu ou en discontinu.

Le lavage à l'eau peut être ainsi effectué en phase liquide à une température comprise entre 5°C et 100°C, sous pression autogène ou sous une pression créée par un gaz inerte tel que l'azote. Le poids d'eau utilisé représente généralement de 0,01 à 1 fois le poids de l'oxydat à laver et de préférence de 0,05 à 0,5 fois ce poids.

Les catalyseurs d'hydrogénation pour l'étape a) sont de préférence le platine, le palladium et le rhodium. lls peuvent être déposés sur des supports tels que silices, alumines, charbons ou aluminosilicates. Le catalyseur peut se présenter sous forme de poudre ou de granulés. La réaction d'hydrogénation est mise en oeuvre en discontinu, par exemple dans autoclave, ou de préférence en continu, par exemple dans une colonne à lit catalytique supporté, ou encore en semi-continu.

L'étape d'hydrogénation a) peut être mise en oeuvre sous une pression d'hydrogène égale à la pression atmosphérique, mais la cinétique de la réaction est alors lente et il y a un risque d'apparition de réactions secondaires dues à un manque d'hydrogène.

C'est pourquoi l'on préfère opérer sous une pression d'hydrogène de 0.5-5 MPa (5 à 50 bar) en température et encore plus préférentiellement de 1-3 MPa (10 à 30 bar). On pourrait opérer sous une pression supérieure à 5 MPa (50 bar), mais sans que cela génère des avantages spécifiques, car la cinétique de la réaction d'hydrogénation est suffisamment élevée dans les zones définies précédemment, notamment dans la zone préférentielle, et en particulier lorsque le catalyseur utilisé est le palladium.

Dans le cas où l'on effectue la réaction d'hydrogénation en continu, on utilise un réacteur d'hydrogénation à lit catalytique supporté, alimenté en une solution aqueuse ne contenant de préférence pas plus de 3 % de peroxydes en poids par poids et de façon encore plus préférée pas plus de 2 % de peroxydes.

L'étape b) d'oxydation par l'acide nitrique est mise en oeuvre en utilisant une solution aqueuse d'acide nitrique possédant un titre pondéral compris entre 40 % et 65 % et de préférence entre 55 % et 60 %.

On utilise généralement un excès molaire d'acide nitrique par rapport aux fonctions alcool à oxyder. En pratique, on met en oeuvre de 4 à 8 moles d'acide nitrique par mole de fonction alcool contenue dans la solution à traiter.

L'oxydation peut être conduite en présence ou non d'un catalyseur, tel qu'un catalyseur à base de vanadium, éventuellement associé à du cuivre, du cobalt, du titane, du nickel, du chrome, du molybdène ou du cérium.

Parmi ces catalyseurs, on préfère l'acide vanadique et les métavanadates de sodium et d'ammonium. En pratique, ce catalyseur est mélangé à la solution aqueuse d'acide nitrique utilisée, à raison de 0,02 mole à 0,2 mole de métal par litre de solution acide, quelle que soit sa concentration.

De manière inattendue, il a été mis en évidence que la réaction d'oxydation pouvait être effectuée en l'absence de catalyseur métallique, tel que cité précédemment, sans pour autant que soit affecté le rendement en acide adipique obtenu. En outre, on peut ainsi obtenir en fin de procédé des diacides aliphatiques exempts de métaux, ce qui est particulièrement intéressant pour certaines de leurs utilisations ultérieures.

Afin d'éviter un déroulement incontrôlé de la réaction d'oxydation, on introduit progressivement la solution déperoxydée dans la solution aqueuse d'acide nitrique, en opérant à une température relativement basse, par exemple entre 15°C et 50°C.

Un des avantages du procédé de l'invention, provenant essentiellement du fait que le mélange soumis à l'oxydation par l'acide nitrique ne contient pratiquement pas de peroxydes, est précisément de pouvoir réaliser cette oxydation à une température de 15°C à 50°C et de préférence entre 30°C et 45°C.

Ces intervalles de température ne sont pas critiques. Cependant, si la température est supérieure à 50°C, par exemple de l'ordre de 90°C, le procédé peut néanmoins être mis en oeuvre, mais le rendement en acide adipique baisse. Si la température est inférieure à 15°C, il peut y avoir une précipitation d'acide adipique.

En fin d'étape d'oxydation, il peut être judicieux de monter temporairement la température pour parfaire la réaction et éliminer certains sous-produits indésirables, tels que par exemple l'acide oxalique. La température à laquelle peut être effectuée cette opération de finition se situe entre 50°C et 100°C et de préférence entre 70°C et 90°C et sa durée peut varier de quelques minutes à plusieurs heures, par exemple entre 15 minutes et 2 heures.

L'acide adipique peut être séparé du mélange réactionnel obtenu après la réaction d'oxydation selon les procédés usuels, tels que par exemple la cristallisation consistant en un refroidissement du mélange et une filtration de l'acide adipique précipité.

L'acide adipique ainsi séparé est ensuite recristallisé selon les techniques habituelles afin d'obtenir un acide adipique de pureté suffisante pour être utilisé dans la fabrication de polyamide-6,6, notamment pour textiles.

Le filtrat obtenu après précipitation de l'acide adipique peut être évaporé à sec afin de conduire à un mélange fondu de diacides, essentiellement un mélange d'acides succinique, glutarique et adipique, avantageusement exempt de métaux, si l'étape b) d'oxydation a été conduite selon une variante préférentielle sans catalyseur. A titre d'exemple, le filtrat peut être plus précisément traité selon la technique décrite dans le brevet FR-A-2 111 003.

Une variante particulièrement avantageuse de traitement du filtrat permet de préparer des mélanges des diacides indiqués précédemment d'excellente qualité notamment en ce qui concerne leur coloration et leur pureté. Elle consiste à chauffer le mélange brut contenant les acides succinique, glutarique et adipique, à l'état fondu, pendant une durée de quelques minutes à plusieurs heures. La température à laquelle est effectué le chauffage peut varier par exemple de 140°C à 220°C. La durée est généralement comprise entre 30 minutes et 15 heures, sans que ces valeurs soient à considérer comme critiques. Le traitement thermique ci-avant est suivi par une distillation du mélange des diacides tel que décrit dans le brevet FR-A-2 111 003. Les mélanges ainsi obtenus sont constitués généralement pour plus de 90 % de leur poids par les acides succinique, glutarique et adipique, les proportions relatives de ces diacides variant en particulier selon la température à laquelle a été refroidi le mélange réactionnel issu de l'étape d'oxydation, pour la précipitation d'acide adipique.

Ces mélanges de diacides peuvent être utilisés notamment sous la forme de leurs esters, essentiellement d'alkyle, tels que méthyle, éthyle, propyle, butyle, pentyle, hexyle, comme solvants dans diverses applications. lls peuvent également servir de plastifiants sous la forme de leurs esters plus lourds, comme les esters d'octyle. Ils peuvent aussi servir d'acidifiants dans l'industrie textile ou l'industrie du cuir.

Les exemples qui suivent illustrent la présente invention.

### EXEMPLE 1

La solution de départ est une solution aqueuse de lavage d'une solution cyclohexanique contenant des hydroperoxydes, provenant d'une réaction d'oxydation du cyclohexane par de l'air appauvri en oxygène, en phase liquide et sans catalyseur, ladite réaction d'oxydation ayant été suivie d'une concentration du mélange réactionnel final.

La solution aqueuse a une teneur en matières organiques (notamment acide hydroxy-6 hexanoïque, acide adipique, acide succinique, acide glutarique, semialdéhyde adipique, esters, peroxydes) de 19,5 % en poids par poids dont 9,7 % de peroxydes.

### Etape a) d'hydrogénation

2500 g de cette solution sont soumis à une hydrogénation, par alimentation continue à raison de 2 litres/heure, à 25°C sous une pression de 1MPa (10 bar) d'hydrogène, dans un autoclave agité, en présence de 1 % en poids, par rapport au poids total de ladite solution, d'un catalyseur en poudre, comprenant 10 % en poids de Pd métal déposé sur du carbone.

En fin d'alimentation, on dégaze l'autoclave et on soutire 2805 g de mélange réactionnel comprenant 15,4 % de matières organiques lourdes (432 g).

On concentre 2525 g de cet hydrogénat pour obtenir 735 g de concentrat titrant 52,5 % en poids de matières organiques lourdes.

### Etape b) d'oxydation

On engage 669,5 du concentrat déperoxydé précédent à l'étape d'oxydation nitrique.

L'oxydation est réalisée en l'absence de catalyseur, dans un appareillage comprenant un réacteur agité.

On introduit 2010 g d'acide nitrique à 58 % en poids par poids dans le réacteur, puis on coule en 2 h, tout en maintenant la température du mélange réactionnel à 40°C, les 669,5 g de concentrat. On élève ensuite la température à 90°C et on maintient cette température pendant 1 h. Ensuite on refroidit jusqu'à température ambiante le mélange réactionnel et on dégaze l'appareillage. On filtre le précipité d'acide adipique (161,4 g après séchage).

Après recristallisation de cet acide adipique, on obtient un produit de très bonne qualité, convenant pour la fabrication de polyamide-6,6.

Le filtrat est concentré à sec pour conduire à un mélange comprenant :
- 52,5 g d'acide adipique
- 83,8 g d'acide glutarique
- 40,2 g d'acide succinique.

Ce mélange est chauffé pendant 10 h à 150°C, puis il est distillé à l'aide d'un évaporateur à film.

Le mélange distillé obtenu se présente sous la forme d'écailles de couleur blanche à jaune très pâle à l'oeil (coloration selon l'indice Gardner de 6).

### EXEMPLE 2

En opérant comme dans l'étape a) de l'exemple 1, on hydrogène une solution aqueuse de lavage ayant une teneur pondérale en matières organiques de 13,85 % dont 10,1 % sont constitués par des peroxydes.

On réalise trois opérations successives en injectant au total 6000 g de solution aqueuse et en hydrogénant à 25°C sous 1MPa (10 bar) d'hydrogène en présence de 1 % en poids par rapport au poids de la solution soumise à l'hydrogénation de catalyseur, constitué par du Pt déposé à 5 % sur du noir de carbone. Chaque hydrogénation dure une heure.

On soutire l'hydrogénat qui titre 13,2 % en poids de matières organiques lourdes (792 g).

Sur 5781 g de cet hydrogénat, on opère une concentration pour obtenir 1386 g de concentrat contenant 755 g de matières organiques lourdes.

On effectue comme décrit dans l'exemple 1, l'oxydation nitrique de 410 g de ce concentrat à 40°C, dans 2002 g d'acide nitrique à 58 %.

Après refroidissement et filtration du précipité formé, on obtient 82,6 g d'acide adipique (poids à sec).

### EXEMPLE 3

La solution de départ est une solution aqueuse de lavage d'une solution cyclohexanique contenant des hydroperoxydes, provenant d'une réaction d'oxydation du cyclohexane par de l'air appauvri en oxygène, en phase liquide et sans catalyseur, ladite réaction d'oxydation ayant été suivie d'une concentration du mélange réactionnel final.

La solution aqueuse a une teneur en matières organiques (notamment acide hydroxy-6 hexanoïque, acide adipique, acide succinique, acide glutarique, semialdéhyde adipique, esters, peroxydes) de 15,1 % en poids par poids dont 10,3 % de peroxydes.

Cette solution est hydrogénée en continu dans un réacteur à colonne à lit catalytique à ruissellement. Le catalyseur est constitué par des granulés en graphite contenant 0,5 % en poids de Pd.

La solution est prédiluée a l'aide de solutions aqueuses déjà hydrogénées, afin que la concentration soit de 1,57 % en peroxydes au sommet du réacteur d'hydrogénation. La pression d'hydrogène est de 2MPa (20 bar) et la température de 25°C.

En pied de réacteur, il n'y a plus de peroxydes.

On effectue ensuite les opérations de concentration et d'oxydation nitrique, sur une partie de l'hydrogénat, comme indiqué dans l'exemple 1. On obtient de l'acide adipique qui, après recristallisation, est un produit de très bonne qualité, convenant pour la fabrication de polyamide-6,6.

## Revendications

1. Procédé de préparation de diacides aliphatiques à partir des eaux de lavage provenant d'un procédé d'oxydation du cyclohexane et contenant des peroxydes, **caractérisé en ce que** l'on effectue successivement les étapes suivantes :
a) - on déperoxyde au moins partiellement lesdites eaux de lavage, en les soumettant à une hydrogénation catalytique à une température comprise entre 0°C et 100°C, en présence d'au moins un métal choisi dans le groupe du platine ;
b) - on oxyde à l'aide d'acide nitrique les produits contenus dans les eaux de lavage après déperoxydation.

2. Procédé selon la revendication 1, **caractérisé en ce que** le métal du groupe du platine est choisi parmi le platine, le palladium, le rhodium, le ruthénium, l'iridium et l'osmium.

3. Procédé selon l'une des revendications 1 ou 2, **caractérisé en ce que** l'hydrogénation catalytique utilisée pour la déperoxydation de l'étape a) est effectuée à une température de 20°C à 80°C et sous une pression d'hydrogène de 0.5-5 MPa (5 à 50 bar) et plus préférentiellement de 1-3 MPa (10 à 30 bar).

4. Procédé selon l'une des revendications 1 à 3, **caractérisé en ce qu'**entre les étapes a) et b), on effectue une concentration de l'hydrogénat obtenu en a), par élimination d'une partie de l'eau qu'il contient.

5. Procédé selon l'une des revendications 1 à 4, **caractérisé en ce que** les eaux de lavage auxquelles s'appliquent le procédé de l'invention proviennent de tout procédé d'oxydation du cyclohexane par l'air, l'air enrichi ou l'air appauvri en oxygène, dans lequel les oxydats sont lavés selon les techniques conventionnelles de lavage en phase liquide, l'opération pouvant être conduite en continu ou en discontinu.

6. Procédé selon la revendication 5, **caractérisé en ce que** le lavage à l'eau est effectué en phase liquide à une température comprise entre 5°C et 100°C, sous pression autogène ou sous une pression créée par un gaz inerte tel que l'azote, le poids d'eau utilisé représentant de 0,01 à 1 fois le poids de l'oxydat à laver et de préférence de 0,05 à 0,5 fois ce poids.

7. Procédé selon l'une des revendications 1 à 6, **caractérisé en ce que** l'étape a) est effectuée en continu dans un réacteur d'hydrogénation à lit catalytique et **en ce que** le réacteur est alimenté avec une solution aqueuse ne contenant pas plus de 3 % de peroxydes en poids et de façon préférée pas plus de 2 % de peroxydes.

8. Procédé selon l'une des revendications 1 à 7, **caractérisé en ce que** l'étape b) d'oxydation par l'acide nitrique est mise en oeuvre en utilisant une solution aqueuse d'acide nitrique possédant un titre pondéral compris entre 40 % et 65 % et de préférence entre 55 % et 60 %.

9. Procédé selon l'une des revendications 1 à 8, **caractérisé en ce que** l'étape b) d'oxydation par l'acide nitrique est mise en oeuvre en utilisant un excès molaire d'acide nitrique par rapport aux fonctions alcool à oxyder, de préférence de 4 à 8 moles d'acide nitrique par mole de fonction alcool contenue dans la solution à traiter.

10. Procédé selon l'une des revendications 1 à 9, **caractérisé en ce que** l'étape b) d'oxydation est conduite en présence d'un catalyseur, tel qu'un catalyseur à base de vanadium, éventuellement associé à du cuivre, du cobalt, du titane, du nickel, du chrome, du molybdène ou du cérium.

11. Procédé selon l'une des revendications 1 à 9, **caractérisé en ce que** l'étape b) d'oxydation est conduite en l'absence de catalyseur.

## Claims

1. Process for the preparation of aliphatic diacids from washing waters derived from a process for the oxidation of cyclohexane and containing peroxides, **characterized in that** the following steps are successively carried out:
a) the said washing waters are at least partially deperoxidized by subjecting them to catalytic hydrogenation at a temperature of between O°C and 100°C, in the presence of at least one metal chosen from the platinum group;
b) the products contained in the washing waters after deperoxidation are oxidized with nitric acid.

2. Process according to Claim 1,15**characterized in that** the metal from the platinum group is chosen from platinum, palladium, rhodium, ruthenium, iridium and osmium.

3. Process according to either of Claims 1 and 2, **characterized in that** the catalytic hydrogenation used for the deperoxidation in step a) ïs carried out at a temperature of from 20°C to 80*C and under a hydrogen pressure of from 0,5 to 5 MPa (5 to 50 bar) at temperature, and more preferably from 0,1 to 0,3 MPa (10 to 30 bar).

4. Process according to one of Claims 1 to 3, **characterized in that** between steps a) and b), the hydrogenate obtained in a) is concentrated by elimination of some of the water contained thereïn.

5. Process according to one of Claims 1 to 4, **characterized in that** the washing waters to which the process of the invention is applied originate from any process of oxidation of cyclohexane with air, oxygen-enriched air or oxygen-depleted air, in which the oxidates are washed according to the conventional techniques of liquid-phase washing, it being possible for the operation to be carried out continuously or non-continuously.

6. Process according to Claim 5, **characterized in that** the washing with water is carried out in the liquid phase at a temperature of between 5°C and 100°C, under the autogenous pressure or under a pressure created by an inert gas such as nitrogen, the weight of water used representing from 0.01 to 1 times the weight of the oxidate to be washed, and preferably from 0.05 to 0.5 times this weight.

7. Process according to one of Claims 1 to 6, **characterized in that** step a) is carried out continuously in a hydrogenation reactor with a catalytic bed and **in that** the reactor is supplied with an aqueous solution containing not more than 3% peroxides, on a weight basis, and preferably not more than 2% peroxides.

8. Process according to one of Claims 1 to 7, **characterized in that** step b) of oxïdation with nitric acid is carried out using an aqueous nitric acid solution having a weight titre of between 40% and 65% and preferably between 55% and 60%.

9. Process according to one of Claims 1 to 8, **characterized in that** step b) of oxidation with nitric acid is carried out using a molar excess of nitric acid relative to the alcohol functions to be oxidized, preferably of from 4 to 8 mol of nitric acid per mole of alcohol function contained in the solution for processing.

10. Process according to one of Claims 1 to 9, **characterized in that** step b) of oxidation is carried out in the presence of a catalyst, such as a vanadium-based catalyst, optionally combined with copper, cobalt, titanium, nickel, chromium, molybdenum or cerium.

11. Process according to one of Claims 1 to 9, **characterized in that** step b) of oxidation is carried out in the absence of a catalyst.

## Patentansprüche

1. Verfahren zur Herstellung von aliphatischen Disäuren aus Waschwässern, die einem Oxidationsverfahren von Cyclohexan entstammen und Peroxide enthalten, **dadurch gekennzeichnet, daß** man nacheinander die folgenden Stufen durchführt:
a) man deperoxidiert zumindest teilweise die Waschwässer, indem man sie einer katalytischen Hydrierung bei einer Temperatur zwischen 0°C und 100°C in Gegenwart von zumindest einem Metall, ausgewählt aus der Platingruppe, unterzieht;
b) man oxidiert die in den Waschwässern nach der Deperoxidation erhaltenen Produkte mit Salpetersäure.

2. Verfahren gemäß Anspruch 1, **dadurch gekennzeichnet, daß** das Metall der Platingruppe ausgewählt wird unter Platin, Palladium, Rhodium. Ruthenium, Iridium und Osmium.

3. Verfahren gemäß einem der Ansprüche 1 oder 2, **dadurch gekennzeichnet, daß** die für die Deperoxidation von Stufe a) angewandte katalytische Hydrierung bei einer Temperatur von 20 bis 80°C und unter einem Wasserstoffdruck von 0,5 bis 5 MPa (5 bis 50 bar) und vorzugsweise von 1 bis 3 MPa (10 bis 30 bar) durchgeführt wird.

4. Verfahren gemäß einem der Ansprüche 1 bis 3, **dadurch gekennzeichnet, daß** man zwischen den Stufen a) und b) eine Konzentrierung des in Stufe a) erhaltenen Hydrierungsprodukts durch Eliminierung eines Teils des Wassers, das dieses enthält, durchführt.

5. Verfahren gemäß einem der Ansprüche 1 bis 4, **dadurch gekennzeichnet, daß** die WaschWässer auf die man das erfindungsgemäße Verfahren anwendet, einem beliebigen Verfahren zur Oxidation von Cyclohexan mit Luft, mit Sauerstof angeraichener Luft oder mit an Sauerstoff verarmter Luft, worin die Oxidationsprodukte entsprechend üblicher Techniken des Waschens in flüssiger Phase gewaschen werden und wobei die Maßnahme kontinuierlich oder diskontinuierlich durchgeführt werden kann, entstammen.

6. Verfahren gemäß Anspruch 5. **dadurch gekennzeichnet, daß** das Waschen mit Wasser in flüssiger Phase bei einer Temperatur zwischen 5°C und 100°C unter autogenem Druck oder unter einem Druck, erzeugt durch ein Inertgas wie Stickstoff, durchgeführt wird, wobei das Gewicht des verwendeten Wassers das 0.01 bis 1-fache des Gewichts des zu waschenden Oxidationsprodukts und vorzugsweise das 0,05 bis 0.5-fache dieses Gewichts beträgt.

7. Verfahren gemäß einem der Ansprüche 1 bis 6, **dadurch gekennzeichnet, daß** die Stufe a) kontinuierlich in einem Hydrierungsreaktor mit Katalysatorbett durchgeführt wird und daß der Reaktor mit einer wäßrigen Lösung beschickt wird, die nicht mehr als 3 Gew.-% Peroxide, vorzugsweise nicht mehr als 2% Peroxide enthält.

8. Verfahren gemäß einem der Ansprüche 1 bis 7, **dadurch gekennzeichnet, daß** die Stufe b) der Oxidation mit Salpetersäure unter Verwendung einer wässrigen Salpetersäurelösung durchgeführt wird, die einen Gewichtstiter zwischen 40 und 65%, vorzugsweise zwischen 55 and 60% besitzt.

9. Verfahren gemäß einem der Ansprüche 1 bis 8, dadurch gckennzeichnet, daß die Stufe b) der Oxidation mit Salpetersäure unter Verwendung eines molaren Überschusses an Salpetersäure in Bezug auf die zu oxidierenden Alkoholfunktionen, vorzugsweise unter Verwendung von 4 bis 8 Mol Salpetersäure je Mol in der zu behandelnden Lösung enthaltenen Alkoholfunktion durchgeführt wird.

10. Verfahren gemäß einem der Ansprüche 1 bis 9, **dadurch gekennzeichnet, daß** die Stufe b) der Oxidation in Gegenwart eines Katalysators, wie eines Katalysators auf Basis von Vanadium, gegebenenfalls in Assoziation mit Kupfer, Kobalt, Titan, Nickel, Chrom, Molybdän oder Cer durchgeführt wird.

11. Verfahren gemäß einem der Ansprüche 1 bis 9, **dadurch gekennzeichnet, daß** die Stufe b) der Oxidation in Abwesenheit eines Katalysators durchgeführt wird.
